# EUROPEAN PATENT SPECIFICATION

(11) **EP 3 826 559 B1**
(45) Date of publication and mention of the grant of the patent: **30.09.2026**
(21) Application number: 19840272.9
(22) Date of filing: 24.07.2019
(51) Int. Cl.: G06T 12/30, G06T 7/73, A61B 17/60, A61B 17/62, A61B 17/66

(54) **METHODS AND SYSTEMS OF REGISTERING A RADIOGRAPHIC IMAGE AND A 3D MODEL OF AN EXTERNAL FIXATION DEVICE**
VERFAHREN UND SYSTEME ZUR AUFNAHME EINES RÖNTGENBILDES UND EINES 3D-MODELLS EINER EXTERNEN FIXIERVORRICHTUNG
PROCÉDÉS ET SYSTÈMES D'ENREGISTREMENT D'UNE IMAGE RADIOGRAPHIQUE ET D'UN MODÈLE 3D D'UN DISPOSITIF DE FIXATION EXTERNE

(30) Priority: 24.07.2018 US 201862702378 P
(43) Date of publication of application: 02.06.2021
(73) Proprietor: Arthrex, Inc., Naples, FL 34108-1945 (US)
(72) Inventor: MULLANEY, Michael W., Naples, Florida 34113 (US)
(74) Representative: Lohr, Jöstingmeier & Partner Patent- und Rechtsanwälte mbB
(86) International application number: PCT/US2019/043326
(87) International publication number: WO 2020/023686

(56) References cited:
- US-A1- 2017 323 443
- US-A1- 2017 323 443
- US-A1- 2018 144 501
- US-B2- 9 642 649
- US-B2- 9 642 649
- BROWBANK I ET AL: "ROBOTIC-ASSISTED INTERNAL FIXATION OF HIP FRACTURES: A FLUOROSCOPY-BASED INTRAOPERATIVE REGISTRATION TECHNIQUE", PROCEEDINGS OF THE INSTITUTION OF MECHANICAL ENGINEERS.JOURNAL OF ENGINEERING IN MEDICINE. PART H, MECHANICAL ENGINEERING PUBLICATIONS LTD, LONDON, GB, vol. 214, no. PART H02, January 2000 (2000-01-01), pages 165 - 179, XP000954513, ISSN: 0954-4119, DOI: 10.1243/0954411001535336

## Description

### FIELD OF THE INVENTION

The present disclosure generally relates to systems and methods for registration of a two-dimensional image (e.g., a radiographic image) and a three-dimensional model of a fixation device (e.g., an external fixation device) for deformity and/or orthopedic analysis and/or correction. The systems and methods determine the relative position and orientation/pose of an image of anatomical structure of interest (e.g., two or more bone segments) and a known fixation device (e.g., an external fixation device, such as a hexapod) secured to the anatomical structure of interest relative to the fixation device. In some embodiments, the systems and methods determine the focal point of the image and account for perspective distortion in the image, and create a coordinate transformation matrix)

The methods and systems of the disclosure enable generation of a three-dimensional computer model of bone segments and a fixation device (e.g., an external fixation device) for the planning of movement of the bone segments into desired locations via the fixation device. For example, through operations on the model, desired position and orientation/pose of the bone segments and adjustments of the fixation device to achieve such desired placement can be determined quickly and accurately regardless of the initial configuration of the fixation device. The operations required to create the desired position and orientation/pose of the bone segments may then be enacted on the corresponding fixation device and bone segments to achieve the desired position and orientation/pose. However, other devices other than external fixation device may be utilized with the system and methods.

### BACKGROUND

The correction of orthopedic deformities usually involves at a minimum a pair of x-ray radiographs. Typically, these radiographs of the patient are attempted to be taken along the conventional lines of an anterior to posterior (AP) direction as well as a medial to lateral (ML) direction, or along other orthogonal or known vantage points (or known difference between vantage points). In accordance with convention, the AP and ML radiographs are taken or assumed to be orthogonal to each other in patient space (the patient space being defined as having the X axis aligned from right to left, the Y axis aligned from anterior to posterior, and the Z axis aligned with inferior to superior). Measurements are made, and deformity axes and points are annotated, within the pair of radiographs. These measurements and annotations within the pair of radiographs are then compared or otherwise used to reconstruct a 3-dimensional representation of the deformity in order that the deformity can be manipulated by some means to correct the condition.

However, inaccuracy in the vantage points of the pair of radiographs, and their spatial relationship to each other, results in an inaccurate representation of the patient and objects coupled thereto contained in the images (i.e., artifacts shown in the images). Radiographs and other patient imaging techniques do not produce perfect images of the artifacts contained within those images. The relationship between the artifacts shown in an image and the actual objects being imaged is one of perspective such that those objects that lie closer to the image have a smaller amount of magnification than those that lie further away. In addition, the vantage points of the images are not perfectly aligned with conventional lines/vantage points (and thereby a pair of images are not truly orthogonal) as the arrangement of the patient with respect to the focal point of the imager is a manual operation. By not accounting for these aspects/inaccuracies of the images, 3D reconstructions of the patient and/or objects coupled thereto contained in the images are not true representations thereof.

As a result, systems and methods that account for the uncertainties/inaccuracies of images of a portion of patient and objects coupled thereto, and construct a true 3D model thereof, are needed. Further, systems and methods that determine the position and pose/orientation of an image of a portion of patient and objects coupled thereto with respect to the objects, on a per-image basis, are needed.

While certain aspects of conventional technologies have been discussed to facilitate disclosure of Applicant's inventions, the Applicant in no way disclaims these technical aspects, and it is contemplated that their inventions may encompass one or more conventional technical aspects.

In this specification, where a document, act or item of knowledge is referred to or discussed, this reference or discussion is not an admission that the document, act or item of knowledge or any combination thereof was, at the priority date, publicly available, known to the public, part of common general knowledge, or otherwise constitutes prior art under the applicable statutory provisions; or is known to be relevant to an attempt to solve any problem with which this specification is concerned.

US 9 642 649 B2 describes methods of orthopedic fixation and imagery analysis. Images of first and second bone segments attached to a fixation apparatus are captured. Fixator elements identified in the images can be used to obtain imaging scene parameters. Bone elements identified in the images can be used with the imaging scene parameters to reconstruct a three dimensional representation of positions and/or orientations of the first and second bone segments with respect to the fixation apparatus.

US 2017/323443 A1 describes methods, hardware, and software to transform 2D anatomical X-ray images into 3D renderings for surgical preparation. X-ray images of a body part are identified by camera model. A contour is extracted from the X-ray. Each anatomical region of the contour is assigned 2D anatomical values. A separate 3D template for the body part is modified to match the X-ray image by extracting silhouette vertices from the template and their projections. The template is aligned with the x-ray image and projected on an image plane to obtain a 2D projection model. The template is modified to match the anatomical values by comparing the projection with the corresponding anatomical values. Best matching points on the contour for extracted silhouette vertex projections are identified and used to back-project corresponding silhouette vertices. The 3D template is deformed so that its silhouette vertices match the target positions, resulting in a 3D reconstruction for the X-ray image.

### SUMMARY OF THE INVENTION

The present invention may address one or more of the problems and deficiencies of the art discussed above. However, it is contemplated that the invention may prove useful in addressing other problems and deficiencies in a number of technical areas. Therefore, the claimed invention should not necessarily be construed as limited to addressing any of the particular problems or deficiencies discussed herein.

In some embodiments, a radiographic or other image of a portion of a patient and objects coupled thereto contains the shadows of the three-dimensional objects that were posed and positioned/located above/below that image (e.g., the film in a radiographic image) when taken. The apparent source location and orientation of the image source (e.g., x-ray source) with respect to the image that is casting that shadow is unknown.

In an ideal world, the focal point of the imager would be a point source placed at an infinite distance above and centered on the image itself. An ideal representation would result in the shadow in the image being a true two-dimensional projection of the actual three-dimensional object. If two such ideal images/representations are obtained, and it is known the images/representations are orthogonal to each other about a common axis, the two sets of two-dimensional data can be directly utilized to accurately reconstruct a three-dimensional model of the object and its position and pose in space.

However, this is not generally possible given that the current state of the art in patient imagery (e.g., radiographic technology involving plain film radiographs) results in perspective distortion, as discussed above. Additionally, the likelihood that the images (e.g., radiographs) are taken truly orthogonal to both the trajectory of the image source and orthogonal to each other about a common axis, is also quite unlikely given all the variables involved in acquiring those images on the actual imaging machine (e.g., an x-ray machine) with an actual patient being instructed to lie/pose in prescribe ways, for example.

The system and methods of the present disclosure utilize the two main sources of error, focal point position and pose as well as patient orientation, to draw a number of conclusions to, ultimately, correct/account for the actual perspective/vantage point of an image and construct a true three-dimensional model of the objects within the image (and potentially the image itself).

The computer-based systems and methods may consider perspective distortion by determining portions (e.g., radiopaque portions) of an object coupled to the patient that are of a particular shape and potentially size (e.g., spherical elements) and the shadows they casted in a single image (e.g., a single radiographic image) (or on a per-image basis). For example, the system and methods may utilize a multitude of known portions of the object in the image, the shadows of which are present as artifacts in the image, and the known relative shape and size of the portions, and relationship of the portions to each other, to determine the apparent focal point location and pose of the image source (e.g., the x-ray source for a radiographic image).

The system and methods may determine the position and pose of the three-dimensional collection of known portions of the object in the image space using a plurality of closed vector loops through the shadow centers, the centers of the actual portions casting the shadows, and the focal point location of the imager. With the plurality of closed vector loops determined, the system and methods may construct a coordinate transform matrix for the collection of the known three-dimensional portions of the object in a shadow image space (i.e., determine a row dimension, a column dimension and a height dimension).

In a first aspect, this disclosure provides a method according to claim 1 and a system according to claim 12 that utilize known three-dimensional collection of objects, the shadows of which are cast in a 2-dimensional x-ray radiographic space, to determine the actual position and pose of that known collection of objects in a projected, and computer-modeled, three-dimensional space lying above the 2-dimensional radiographic space.

In some embodiments, the methods and systems may utilize perspective distortion to determine relative magnifications to aid in the reconstruction of the three-dimensional projected space. In some embodiments, the methods and systems may reconstruct a model of the actual three-dimensional conditional in a corrected relative spatial arrangement.

Certain embodiments of the presently-disclosed systems and methods may include several features, no single one of which is solely responsible for their desirable attributes. Without limiting the scope of the systems and methods, their more prominent features will now be discussed briefly. After considering this discussion, and particularly after reading the section of this specification entitled "Detailed Description of the Invention," one will understand how the features of the various embodiments disclosed herein provide a number of advantages over the current state of the art.

These and other features and advantages of this invention will become apparent from the following detailed description of the various aspects of the invention taken in conjunction with the appended claims and the accompanying drawings.

### BRIEF DESCRIPTION OF THE DRAWINGS

The present disclosure will hereinafter be described in conjunction with the following drawing figures, which are not necessarily drawn to scale for ease of understanding, wherein the same reference numerals retain their designation and meaning for the same or like elements or aspects throughout the various drawings, and wherein:
FIG. 1 is a perspective view of an exemplary external fixation device coupled to exemplary bone segments;
FIG. 2 is a front view of a two-dimensional radiographic image depicting an exemplary external fixation device coupled to exemplary bone segments;
FIG. 3 is a perspective view of a constructed digital three-dimensional model including a digital two-dimensional radiographic image and a digital three-dimensional model of an external fixation device represented in the model in correct relative positions and poses with respect to a determined focal point of the radiographic image;
FIG. 4 is a flow diagram of an exemplary method of digitally registering a radiographic image and a three-dimensional model of an external fixation device depicted in the radiographic image;
FIG. 5 depicts an exemplary method for digitally constructing a coordinate transformation matrix that identifies the position and pose of a radiographic image relative to a three-dimensional model of an external fixation device depicted in the radiographic image;
FIG. 6 depicts an exemplary computer system that may be utilized to perform aspects of the present disclosure; and
FIG. 7 depicts an embodiment of a computer program product that may incorporate of the present disclosure.

### DETAILED DESCRIPTION OF THE INVENTION

Aspects of the present inventions and certain features, advantages, and details thereof, are explained more fully below with reference to the non-limiting embodiments illustrated in the accompanying drawings. Descriptions of well-known materials, fabrication tools, processing techniques, etc., are omitted so as to not unnecessarily obscure the inventions in detail.

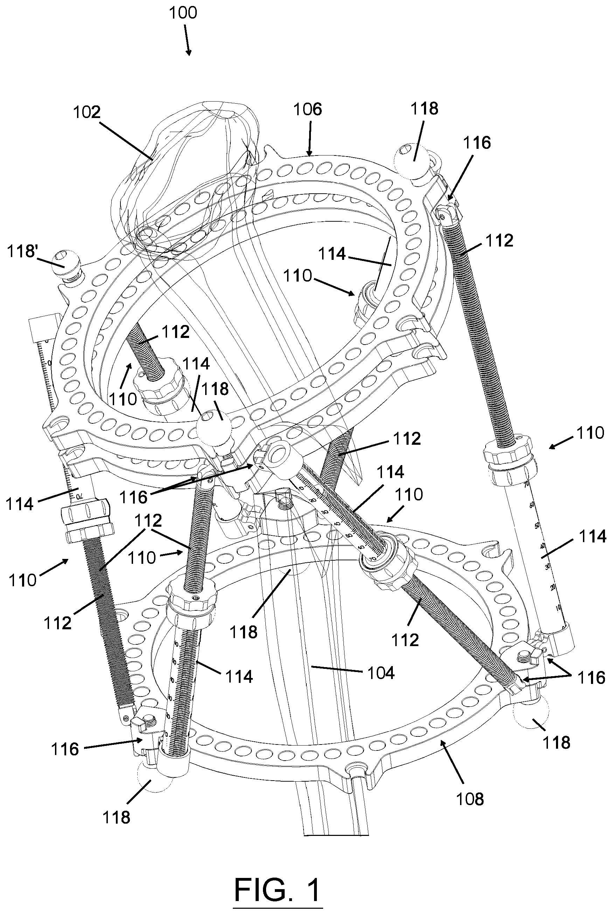

Referring initially to FIG. 1, bodily tissues, for instance first and second bone segments 102, 104, can be aligned and/or oriented to promote union or other healing between the bodily tissues. The alignment and/or orientation of the bodily tissues can be achieved by connecting the bodily tissues to an adjustable fixation apparatus, such as an orthopedic external fixation device or fixator 100. The fixation device can comprise a plurality of discrete fixator platforms or members that remain external to the patient's body, but that are attached to respective discreet bodily tissues, for example with minimally invasive attachment members. By adjusting the spatial positioning of the platforms with respect to each other, the respective bodily tissues attached thereto can be reoriented and/or otherwise brought into alignment with each other, for example to promote union between the bodily tissues during a healing process. The use of external fixation devices in combination with the imagery analysis and positioning techniques described herein can be advantageous.

The fixator members can be connected to each other via adjustment struts or members, the adjustment struts configured to facilitate the spatial repositioning of the platforms with respect to each other. For example, in the illustrated embodiment shown in FIG. 1, the external fixation device 100 comprises a pair of platforms or fixator members in the form of at least one upper ring platform 106 and a lower ring platform 108. The platform rings 106, 108 can be constructed the same or differently. For instance, the platform rings 106, 108 can have diameters that are the same or different. Similarly, the platform rings 106, 108 can be constructed with varying cross sectional diameters, thicknesses, etc. It should be appreciated that the fixator members of the fixation device 100 are not limited to the illustrated upper and lower platform rings 106, 108, and that the fixation device 100 can be alternatively constructed. For example, additional fixator rings can be provided and interconnected with the upper ring platform 106 and/or the lower ring platform 108. It should further be appreciated that the geometries of the platforms are not limited to rings, and that at least one, such as any or all of the platforms can be alternatively constructed using any other suitable geometry.

The first and second bone segments 102, 104 can be rigidly attached to the upper and lower platform rings 106, 108, respectively, with attachment members (not shown) that can be mounted and/or coupled to the platform rings 106, 108. For example, the external fixation device may include attachment rods and/or attachment wires (not shown) that are fixed or coupled to the upper and lower platform 106, 108 (e.g., directly or via a clamp or any other mounting mechanism configuration) and to the first and second bone segments 102, 104, respectively.

The attachment members can be removably mounted to the platform rings 106, 108 at predefined points along the peripheries of the platform rings 106, 108, for example by coupling them to apertures defined by the platforms 106, 108. With respect to each platform 106, 108, the attachment members and/or mounting mechanisms or members coupled therewith can be mounted to the upper surface of the platform, the lower surface of the platform, or any combination thereof. It should be appreciated that the configuration of the attachment members is not limited to the configurations discussed above. For example, any number of attachment members, such as any number of pins, wires or the like, can be used to secure the bone segments 102, 106 and to a respective platform 106, 108 as desired. It should further be appreciated that one or more of the attachment members can be configured to mount or couple directly to/with the platforms 106, 108 without utilizing an intermediary or additional mounting mechanism, or may be configured to mount indirectly to the platforms 106, 108 via a mounting mechanism or with the assistance or aid of a mounting mechanism.

The upper and lower platform 106, 108 of the external fixation device 100 are connected to each other by a plurality of adjustment struts or members 110, as shown in FIG. 1. At least one, such as all, of the adjustment struts can be configured to be adjusted to adjust the spatial positioning and pose of the platforms 106, 108 with respect to each other. For example, in the illustrated embodiment, the upper and lower platform rings 106, 108 are connected to each other with six circumferentially-spaced length adjustable struts 110 that extend therebetween (at least partially). The external fixation device 100 may thereby comprise a hexapod or Stewart platform. It should be appreciated, however, that the construction of the fixation device 100 is not limited to the six struts 110 of the illustrated embodiment, and that more or fewer struts 110 can be utilized as desired.

As shown in FIG. 1, one or more of the length adjustable struts 110 can comprise an axially-elongate threaded rod portion 112 rotatably coupled to one of the first and second platforms 106, 108 via a joint 116, and an axially-elongate barrel portion 114 rotatably coupled to the other of the first and second platforms 106, 108 via another joint 116. The struts 110 are configured such that the threaded rod portion 112 extends within/through at least a portion of the barrel portion 114 in a telescoping manner. As shown in FIG. 1, in some embodiments the struts 110 may be arranged in pairs of oppositely oriented struts 110 coupled to the same portion of the first or second platforms 106, 108.

The threaded rod portion 112 and the barrel portion 114 are threadably coupled such that rotation of at least a portion of the threaded rod portion 112 or the barrel portion 114 with respect to the other portion adjusts the relative axial position therebetween to adjust (i.e., shorten or lengthen) the overall or total axial length of the strut 110. In this way, the struts 100 cab ne adjusted to change or alter the relative position and/or pose/orientation between the first and second platforms 106, 108. In some embodiments, as shown in FIG. 1, the barrel portion 114 may include an adjustment knob that is rotatable about (and potentially translatable along) the (aligned) axes of the threaded rod portion 112 and the barrel portion 114 and is configured to rotate at least a portion of the threaded rod portion 112 or the barrel portion 114 that is threadably coupled to the other portion with respect to the other portion to adjust the total length of the strut assembly 110 (i.e., adjust the telescopic arrangement of the threaded rod portion 112 and the barrel portion 114). However, the threaded rod portion 112 and the barrel portion 114 may include any configuration or arrangement such that the telescopic axial arrangement of the threaded rod portion 112 and the barrel portion 114 can be adjusted to adjust the total length of the strut assembly 110. It should further be appreciated that adjusting the positions of the first and second platforms 106, 108 is not limited to adjusting the lengths of the length adjustable struts 110, and that the positioning of the first and second platforms 106, 108 with respect to each other can be alternatively adjusted, for example in accordance the type and/or number of adjustment members connected to the fixation device 100.

The axial length of each strut assembly 110 can thus be independently adjusted. The adjustable length struts 110 and the universal joints 116 by which they are mounted to the platforms 106, 108, allow the fixation device 100 to function like a Stewart platform, and more specifically like a distraction osteogenesis ring system, a hexapod, or a Taylor spatial frame. As such, by making length adjustments to the struts 110, the spatial positioning of the platforms 106, 108, and thus the spatial positioning of the bone segments 102, 104 coupled thereto, can be altered. For example, in one non-limiting embodiment, varying the length of one or more of the struts 110 may change the relative position and pose of the upper and lower platforms 106, 108, and thus the bone segments 102, 104 coupled thereto, such that longitudinal axes of the bone segments 102, 104 are substantially aligned with each other (e.g., such that their respective ends abut each other, so as to promote union during a healing process for example).

Repositioning of the first and second platforms 106, 108 of the orthopedic external fixation device 100 can be used to correct displacements of angulation, translation, rotation, or any combination thereof, of the bodily tissues 102, 104. The fixation device 100, utilized with the techniques described herein, can correct a plurality of such displacement defects individually or simultaneously.

As shown in FIG. 1, the fixation device 100 may include a plurality of fiducial markers 118 coupled thereto. The fiducial markers 118 may be coupled to the first or second platforms 106, 108 in a fixed relationship to each other, the platforms 106, 108 and at least a portion of the strut assemblies 110. For example, the fiducial markers 118 may be positioned at fixed three-dimensional spatial relationships with respect to at least a portion of one or more proximate strut assembly 110, such as the joint 116 of one or a pair of proximate strut assemblies 110.

The fiducial markers 118 may be of a particular shape and size that differs from that of any other component of the fixation device 100. In this way, the shape and size of the fiducial markers 118 may be unique to the fiducial markers 118. Further, at least one fiducial marker 118' can differ from the other fiducial markers 118. For example, as shown in FIG. 1, one fiducial marker 118' may be of a smaller spherical shape as compared to the other fiducial markers 118. The unique fiducial marker 118' may thereby identify or distinguish the particular platforms 106, 108 that it is coupled to from the other platform 106, 108. For example, the unique fiducial marker 118' may be coupled to the first upper platform 106, and thereby be utilized to identify or deduce the first upper platform 106 from the second lower platform 108, such as in an image of the fixation device 100 (as described below).

As shown in FIG. 1, in some embodiments the fiducial markers 118 may comprise spherical portions that extend from, and/or are positioned proximate or adjacent to, one of the platforms 106, 108. In some embodiments, the fiducial markers 118 may be circumferentially or angularly spaced about the platforms 106, 108. For example, each fiducial markers 118 may be fixedly coupled to the same portion or general area of one of the platforms 106, 108 as a pair of struts 110. As shown in FIG. 1, the fixation device 100 may thereby include three fiducial markers 118 coupled to the first upper platform 106, each fiducial marker 118 position proximate to ends of a pair of strut assemblies 110 coupled to the first upper platform 106 via respective joints 116. As shown in FIG. 1, the fixation device 100 may thereby also include three fiducial markers 118 coupled to the second lower platform 108, each fiducial marker 118 position proximate to ends of a pair of strut assemblies 110 coupled to the second upper platform 108 via respective joints 116.

The fiducial markers 118 are configured to be visible when the fixation device 100 is imaged, such x-rayed. For example, at least the (spherical) outer surface portion of the fiducial markers 118 may be radiopaque. The pre-determined or known locations of the fiducial markers 118 can thereby be used to identify or deduce the identification of the platforms 106, 108, and/or the position and pose of the platforms 106, 108, from an image (e.g., a radiograph/x-ray) of the fixation device 100 as explained further below.

The fiducial markers 118 can be mounted to specific pre-identified or known locations of components of the fixation device 100 prior to imaging thereof (e.g., radiographic imaging thereof), can be imbedded within components of the fixation device 100, or any combination thereof. The marker elements can be configured for enhanced viewability of an image of the fixation device 100 when compared to the viewability of the other components of the fixation device 100. For example, the fiducial markers 118 may be constructed of a different material, such as a radio-opaque material, or may be constructed with geometries that readily distinguish them from other components of the fiducial markers 118 in a radiographic image of the fixation device 100.

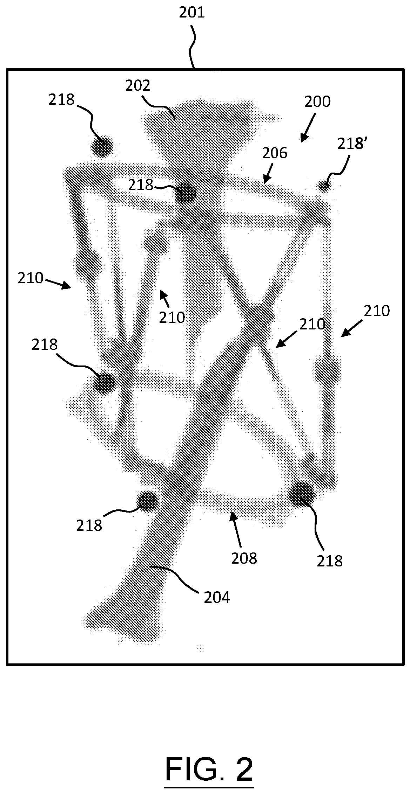

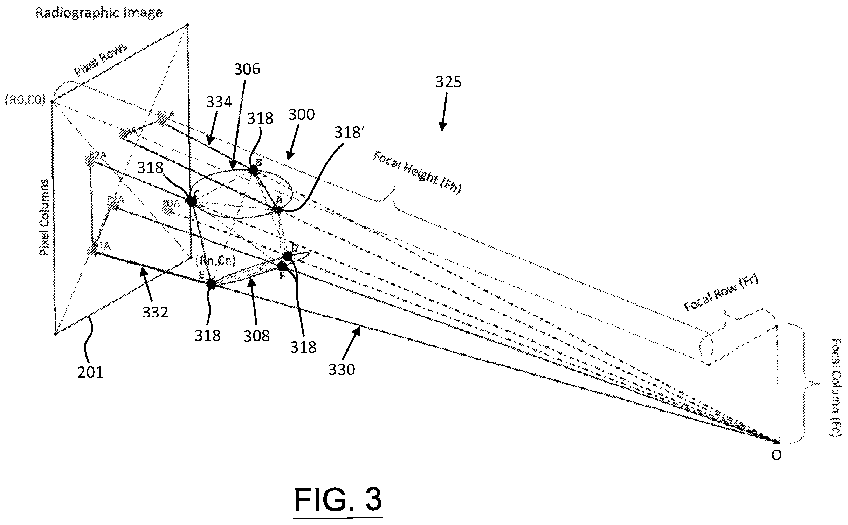

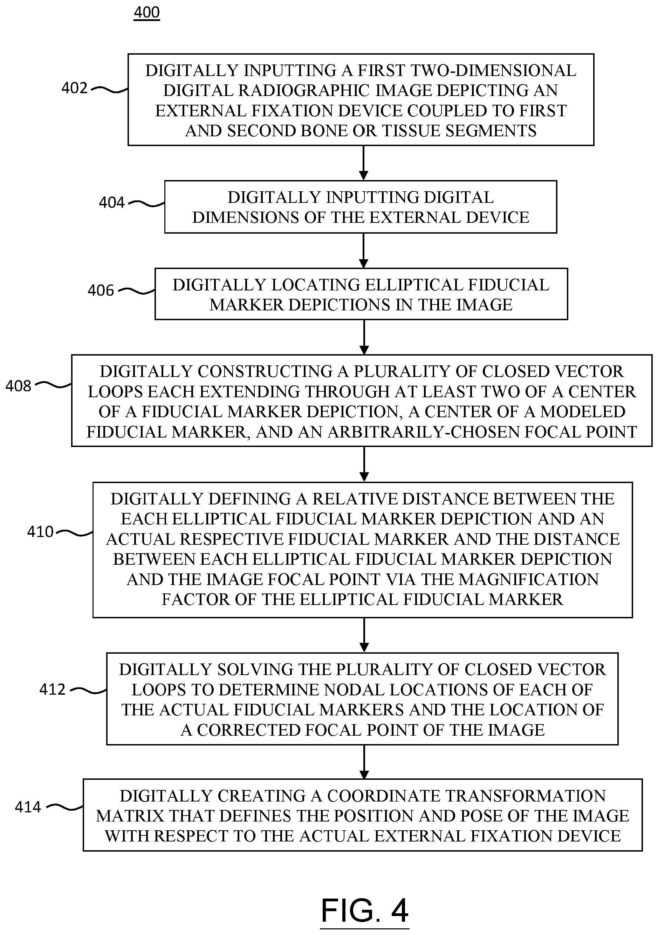

Referring now to FIGS. 2-4, an exemplary method of digitally registering a digital radiographic image 201 depicting an external fixation device (and anatomical structure coupled thereto, such as bone segments or other tissue(s)) and a three-dimensional model 300 of the depicted external fixation device, is illustrated. More specially, a digital radiographic image 201 of patient with an external fixation device coupled to his/her anatomical structures is illustrated in FIG. 2. The digital radiographic image 201 thereby includes a depiction of an external fixation device 200 (corresponding to the external fixation device of the patient) and the anatomical structures (e.g., bone segment or other tissue segments) 202, 204 coupled to platforms 206, 208 of the external fixation device 200. Aspects of a method 400 of digitally registering the radiographic image 201 of FIG. 2 depicting the external fixation device 200 and a three-dimensional model 300 of the external fixation device of the patient (and therefore also the depicted external fixation device 200) is illustrated in the flow chart of FIG. 4. Further, FIG. 3 illustrated a digitally constructed model 325 of the digital radiographic image 201 and a digital three-dimensional model 300 of the external fixation device of the patient and (and therefore also the depicted external fixation device 200 of the radiographic image 201) in a relative position and pose/orientation with respect to the three-dimensional modeled external fixation device 300 constructed via the method 400 of FIG. 4.

The external fixation device of the patient that is imaged, and thereby the external fixation device 200 depicted in the image 201 and the three-dimensionally modeled external fixation device 300 of the three-dimensional model 325, may be an external fixation device that is the same or similar to the external fixation device 100 described above with respect to FIG. 1. For example, the external fixation device of the patient that is imaged, and thereby the external fixation device 200 depicted in the image 201 and the three-dimensionally modeled external fixation device 300 of the three-dimensional model 325, may include one or more like component, aspect, function, process and/or function as compared to the external fixation device 100 of FIG. 1. Therefore, like reference numerals preceded with "2" with respect to the external fixation device 200 depicted in the image 201, and like reference numerals preceded with "3" with respect to the three-dimensionally modeled external fixation device 300, are used to indicate like components, aspects, functions, processes and/or functions to the external fixation device 100 of FIG. 1, and the description above directed thereto equally applies, and is not repeated for brevity and clarity purposes. For example, the external fixation device that is coupled to a patient's anatomy and imaged, and thereby the external fixation device 200 depicted in the image 201 and the modeled external fixation device 300 (depicted in a three dimensional model 325 with the image 201 in a true or corrected relative position and pose, and with respect to a digitally modeled or identified focal point O of the image 201) may be configured as a hexapod including at least a pair of first upper and second lower platforms coupled to at least two bone or tissue segments, respectively, six length-adjustable struts extending between the platforms, and spherical fiducial markers proximate to the ends of pairs of struts on each platform (and one unique fiducial marker that identifies a particular platform, and thereby each platform).

As shown in FIG. 4, at aspect 402, the method 400 may include inputting a first two-dimensional digital radiographic image, such as an image 201 of the patient depicting an external fixation device 200 coupled to first and second bone or tissue segments 202, 204 as shown in FIG. 2. As shown in FIG. 2, the image 201 may include an external fixation device depiction 200 (including fiducial marker depictions 218) and first and second bone or tissue segment depictions 202, 204. The image 201 may be a digital radiographic image or any other two-dimensional image that includes perspective distortion and an unknown, inaccurate or mis-identified viewpoint/focal point. It is noted that the depictions of the fixation device 200 and the first and second bone or tissue segment 202, 204 may be equivalent or similar to shadows of the actual fixation device and bone or tissue segments that is imaged that are cast on the film or other image detection plane when the actual fixation device and bone or tissue segment is positioned between the image detection plane and the focal point. As such, the image 201 comprises inherent projectional distortion.

The image 201 may be digitally input by a user, or the image 201 may be obtained from an imager (not shown). For example, the digital image 201 can be acquired using x-ray imaging, computer tomography, magnetic resonance imaging, ultrasound, infrared imaging, photography, fluoroscopy, visual spectrum imaging, or any combination thereof. The image 201 can be captured from any position and/or orientation with respect to the actual fixation device and bone or tissue segments.

The method 400 may include inputting a plurality of images of the actual fixation device and bone or tissue segments, and thereby including an external fixation device depiction 200 (including fiducial marker depictions 218) and first and second bone or tissue segment depictions 202, 204, that are taken from differing viewpoints or focal points. The method 400 may process each image separately or individually a per image basis, rather than comparing, contrasting or otherwise analyzing the images with respect to each other.

As shown in FIG. 2, an exemplary digitally input image 201 may depict first upper and second lower platforms 206, 208 coupled to at least two bone or tissue segments 202, 204, respectively, six length-adjustable struts 110 extending between the platforms 206, 208, and spherical fiducial markers 218 proximate to the ends of pairs of struts on each platform (and one unique fiducial marker 218' that identifies a particular platform, and thereby each platform).

As shown in FIG. 4, at 404, digital dimensions of the actual external device may be digitally input. For example, a user may enter one or more dimensions of the actual external device that are, or correspond to, the digital dimensions. For example, digital dimensions corresponding to the diameters of the actual fiducial markers, cord distances between the actual fiducial markers on each platform, and the axial lengths of the actual struts may be digitally input. The method 400 may calculate the distance between corresponding fiducial markers at the opposing ends of each strut via a known relationship between the distances and the axial lengths of the actual struts.

Referring to FIG. 3, the method 400 may digitally model or create a three-dimensional model 325 with a three-dimensional modeled external fixation device 300 corresponding to the actual external fixation device relative to the image 201 and an arbitrary focal point O. The three-dimensional modeled external fixation device 300 may consist of a the platforms 306, 308 disposed in space having attached to them the six spherical radiopaque fiducial markers 318 that serve as known shapes A, B, C, D, E, F (via the input digital dimensions), with known (via the input digital dimensions) cord distances AB, BC, CA and DE, EF, and FD therebetween. The fiducial marker shapes A, B, and C are also spaced from fiducial marker shapes D, E, and F at known lengths (via the input digital dimensions). FIG. 3 thus depicts a three-by-three (3x3) fiducial marker configuration, which refers to the three coincident fiducial markers on each of the first and second platforms 306, 308. In this example, the unique fiducial marker 318' denoted by A is smaller than the rest of the fiducial markers 318 (which are all the same size). The unique fiducial marker 318' A differentiates the first platform 306 from the second platform 308 and the rotation of the first platform 306 in image space.

When the fiducial markers of the actual external fixation device are spherical as shown in FIG. 1, the fiducial marker depictions 218 in the image 201 are spherical due to the position of the focal point O of the image 201 and the imaging plane relative to the fiducial markers, as shown in FIG. 2. The elliptical fiducial marker depictions 218 in the image 201 may include outer edges that are relatively sharp, as shown in FIG. 2. At 406, the method 4000 may digitally locate the elliptical fiducial marker depictions 218 in the image 201, as shown in FIG. 4. The method 400 may utilize the shape edges of the elliptical fiducial marker depictions 218, and conclude that the respective fiducial markers that were the sources of the fiducial marker depictions 218 are in fact points located and posed somewhere above the shadows/depictions 218. The method 400 can also conclude that each actual fiducial marker lies on a vector describing the line between the center of each elliptical fiducial marker depiction 218 and the focal point of the source of the image 201, as shown in FIG. 3.

At 408, the method 400 may further determine or relate (or define) the relative distance between the each elliptical fiducial marker depiction 218 and the actual respective fiducial marker and the distance between each elliptical fiducial marker depiction 218 and the image source or focal point O via the magnification factor of each elliptical fiducial marker depiction 218, as shown in FIG. 4. In some embodiments, the method 400 located and evaluated the size and position of the elliptical fiducial marker depictions 218 within the image 201. In some embodiments, the method 400 may utilize a digitally determined minor axis dimension of the fiducial marker depictions 218 in relation to the digitally input actual diameters of the elliptical fiducial marker depiction 218, which may be related to the relative distance between the image 201 and the focal point O and the height along a vector extending therebetween where the actual fiducial marker lies. In some embodiments, the method 400 may utilize the image resolution to determine an initial image scale and relative size of the fiducial marker depictions 218 relative to their digitally input actual size.

For example, the method 400 may digitally identify or measure a diameter of each elliptical fiducial marker depiction 218 (e.g., identify or measure the minor diameter or an average diameter of each elliptical fiducial marker depiction 218, for example) and compare the diameter of each elliptical fiducial marker depiction 218 with the actual diameter of the corresponding actual fiducial marker to digitally determine the magnification factor of the each elliptical fiducial marker depiction 218, and utilize the magnification factor to relate or describe the relative distance between the fiducial marker depictions 218 in the image 201 and the actual fiducial markers and the distance between the fiducial marker depictions 218 and the image source or focal point O. The method 400 may thereby utilize the digitally determined diameters of each elliptical fiducial marker depiction 218 and the digitally input actual diameters of the respective fiducial markers of the actual fixation device to determine or describe the relative distances between the elliptical fiducial marker depictions 218 and the actual fiducial markers as functions of the distance between the fiducial marker depictions 218 and the image source or focal point O. The method may thereby determine an equation, expression or relationship of the distance (e.g., distance along a Z axis extending normally from the image 201, for example) from the image 201 for each actual fiducial marker.

The distances between the each elliptical fiducial marker depiction 218 and the actual respective fiducial marker, by itself, does not indicate the position and pose of the image source or focal point O. The method 400 may utilize the input dimensions related to the distances between the actual fiducial markers to determine the apparent focal point location and pose of image source or focal point O.

The system and methods may determine the position and pose of the actual external fixation device corresponding to the depicted external fixation device 200 in the radiographic image 201 via the digitally modeled or created three-dimensional model 325 with the three-dimensional modeled external fixation device 300 corresponding to the actual external fixation device relative to the image 201 and the arbitrary focal point O, as shown in FIG. 3. As shown in FIG. 3, the focal point O is defined as an arbitrary point floating in space above the image 210.

At 410, the method 400 may define a plurality of constraints by construct a plurality of closed vector loops each extending or passing through at least two of a center of a fiducial marker depiction 218 of the image 201, a center of a modeled fiducial marker 318 of the three-dimensional modeled external fixation device 300, and the arbitrarily-chosen focal point location O, as shown in FIGS. 3 and 4. As a non-limiting example, the method 400 may construct a first closed vector loop 330 of P1A-O-P2A-P1A, a second closed vector loop 332 of B2A-C-E-P1A-B2A, and/or a third closed vector loop 334 of B0A-A-B-B1A-BOA, as shown in FIG. 3.

At 412, the method 400 may solve the plurality of closed vector loops (e.g., such that they collapse on themselves and/or equal zero) to determine the nodal locations A, B, C, D, E, F of each of the actual fiducial markers of the external fixator (in X and Y and Z coordinates from the image 201, for example), and the location of an actual, true or corrected focal point O of the image 201 (in X and Y and Z coordinates from the image 201, for example), as shown in FIG. 4. It is noted that the nodal locations A, B, C, D, E, F of each of the actual fiducial markers of the external fixator and the location of the actual focal point O of the image 201 may be determined (i.e., the problem sufficiently constrained) utilizing a limited number of such closed vector loops. However, relatively more such closed vector loops can be used to statistically improve the results.

Having determined all of the nodal locations A, B, C, D, E, F, and O in image space, at 414, the method 400 may construct a suitable first coordinate transformation matrix (or coordinate transform) for the known (via the input dimensions) actual external fixation device (depicted at least partially by the collection of spherical fiducial markers) relative to the image 201, as shown in FIG. 4. The method 400 can then take the inverse of the coordinate transformation matrix, at 414, to determine a second coordinate transformation matrix that defines or described the position (e.g., X, Y and Z location) and pose (i.e., orientation in X, Y and Z) of the image 201 with respect to the actual external fixation device, as shown in FIG. 4.

In some embodiments, the method 400 may construct a suitable first coordinate transform by determining the cross product of a pair of suitable vectors between the nodes of a common platform to determine a vector normal to both suitable vectors whose origin lies at a node common thereto. For example, the method 400 may take a cross product of ABxAC, and thereby determine a vector normal to both AB and AC whose origin lies at A. The method 400 may then cross the resultant vector with one of the previous suitable vectors to determine an orthogonal coordinate system (i.e., a coordinate transformation matrix) that depicts or describes, in this case, the platform in image space (defined by the respective chosen nodes, such as by ABC in the example above). With the plurality of closed vector loops determined, the method 400 may thereby define a coordinate transform for the collection of the known actual fiducial markers in a shadow image space (i.e., determine a row dimension, a column dimension and a height dimension).

The method 400 can utilize the second coordinate transformation matrix to create the digital three-dimensional model 325 of a three-dimensional model 300 of the actual external fixation and the image 201 in a relative position and pose/orientation with respect to the three-dimensional modeled external fixation device 300, with the viewpoint or camera view of the image focal point O. The method may render the three-dimensional model 300 to display the three-dimensional model 300 to a user so that the user can qualitatively inspect or review the three-dimensional model 300 to ensure that the three-dimensional model 300 constructed via the method 400 is in the correct position and pose with respect to the image 201.

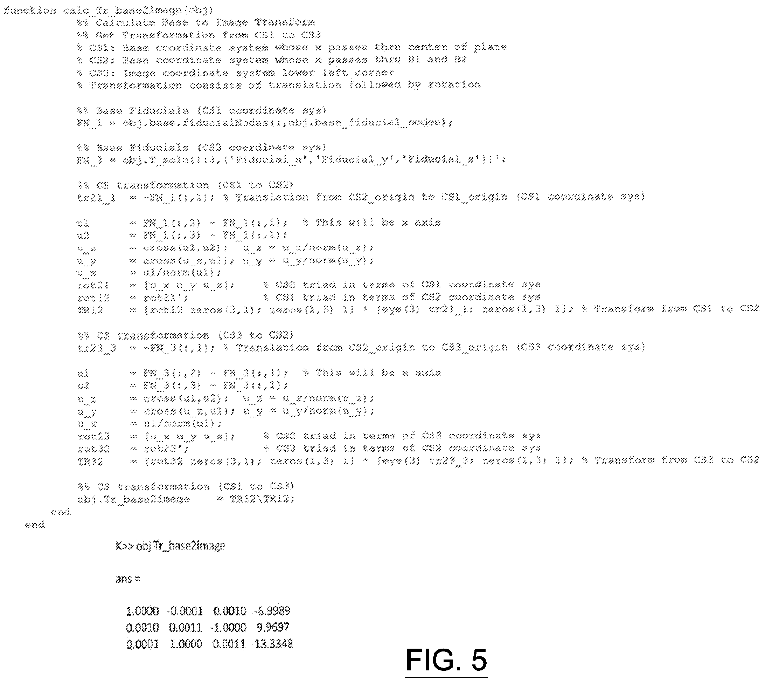

An example of constructing the second coordinate transformation matrix, and a resultant coordinate transformation matrix, for the digitally modeled external fixator 300 and the image 201 of FIG. 3 is depicted in FIG. 5.

With the coordinate system determined, the methods 400 can determine the coordinate transformation between any pair of images within the plurality of images utilizing the collection of known three-dimensional objects in each of the plurality of the radiographic images using a consistent approach in each image. The system and methods may correct for any non-orthogonal or otherwise rotated pairs of images when constructing the true three-dimensional location and pose of the three-dimensional objects, thus accurately describing whatever other annotations or measurements that are made within the radiographic images.

The methods and systems may use the method 400 for each of a plurality of images 201 on an image by image basis to determine a plurality of coordinate systems all describing the same known fiducial marker (external fixation device) in the greater patient space.

As would be evident to one of ordinary skill in the art, the inventions of this disclosure provide significant improvements in the field of external fixation device and anatomical structure computer modeling, including the field of hexapod and bone segment modeling. Further, the inventions of this disclosure provide significant improvements in the field of radiographic imaging, including the field of distortion correction of radiographic images. The inventions of this disclosure also provide significant improvements in the field of external fixation device adjustment prescription determination, including the field of hexapod adjustment prescriptions.

Those having ordinary skill in the art will recognize that aspects of the present invention may be embodied in system(s), method(s) and/or computer program product(s). In some embodiments, aspects of the present invention may be embodied entirely in hardware, entirely in software (for instance in firmware, resident software, micro-code, etc.), or in a combination of software and hardware aspects that may all generally be referred to herein as a "system" and include circuit(s) and/or module(s).

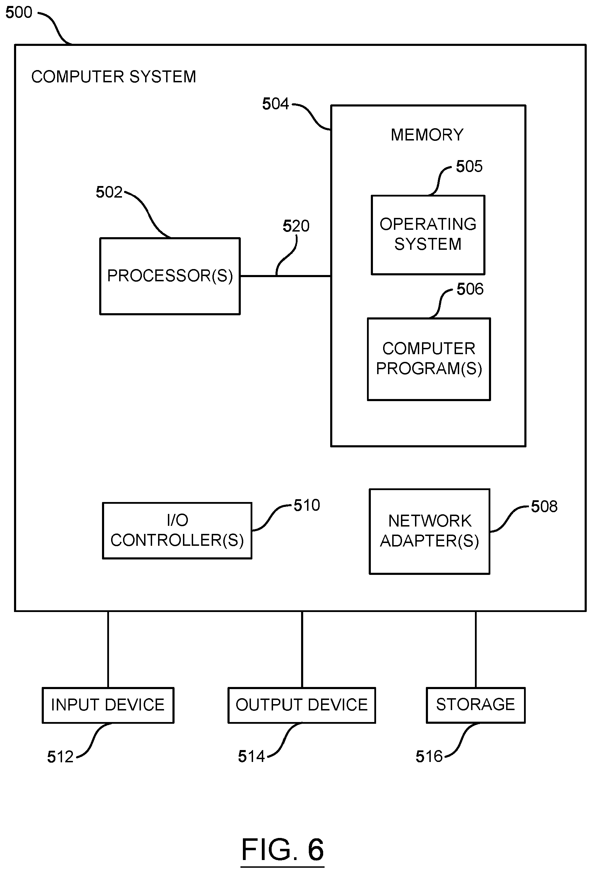

FIG. 6 depicts one example of a computer system to incorporate and use one or more aspects of the present invention. Computer system 500 may be a computer system of an article manufacturing and/or repair facility, such as a computer system used to additively manufacture articles, and/or a computer system for producing data used by an AM apparatus or device to fabricate articles. Computer system 500 of FIG. 6 may be suitable for storing and/or executing program code, such as program code for performing processes described above and includes at least one processor 502 coupled directly or indirectly to memory 505 through, a bus 520. In operation, processor(s) 502 may obtain from memory 505 instructions for execution by the processor(s). Memory 505 may include local memory employed during actual execution of the program code, bulk storage, and cache memories which provide temporary storage of at least some program code in order to reduce the number of times code must be retrieved from bulk storage during program code execution. A non-limiting list of examples of memory 505 includes a hard disk, a random-access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. Memory 505 may include an operating system 505 and one or more computer programs 506, such as one or more programs for execution to perform aspects described herein, such as effecting adjustments to a digital layout of a circuit design.

Input/Output (I/O) devices 512, 514 (such as peripheral devices) may be coupled to the system either directly or through I/O controllers 510. Network adapters 508 may also be coupled to the system to enable the computer system to become coupled to other computer systems through intervening private or public networks. Modems, cable modem and Ethernet cards are just a few of the currently available types of network adapters 508. In one example, network adapters 508 facilitate obtaining data from remote sources to facilitate aspects of the present invention.

Computer system 500 may be coupled to storage 516 (e.g., a non-volatile storage area, such as magnetic disk drives, optical disk drives, a tape drive, etc.), having one or more databases. Storage 516 may include an internal storage device or an attached or network accessible storage. Computer programs in storage 516 may be loaded into memory 505 and executed by a processor 502.

The computer system 500 may include fewer components than illustrated, additional components not illustrated herein, or some combination of the components illustrated and additional components. Computer system 500 may include any computing device, such as a mainframe, server, personal computer, workstation, laptop, handheld computer, smartphone, table, or other mobile device, telephony device, network appliance, virtualization device, storage controller, etc.

In addition, processes described above may be performed by multiple computer systems 500, working in concert as part of a computing environment.

In some embodiments, aspects of the present invention may take the form of a computer program product embodied in computer readable medium(s). The computer readable medium(s) may have embodied thereon computer readable program code. Various computer readable medium(s) or combinations thereof may be utilized. For instance, the computer readable medium(s) may include a computer readable storage medium, examples of which include (but are not limited to) one or more electronic, magnetic, optical, or semiconductor systems, apparatuses, or devices, or any suitable combination of the foregoing. Example computer readable storage medium(s) include, for instance: an electrical connection having one or more wires, a portable computer diskette, a hard disk or mass-storage device, a random access memory (RAM), read-only memory (ROM), and/or erasable-programmable read-only memory such as EPROM or Flash memory, an optical fiber, a portable compact disc read-only memory (CD-ROM), an optical storage device, a magnetic storage device (including a tape device), or any suitable combination of the above. A computer readable storage medium is defined to include a tangible medium that can contain or store program code for use by or in connection with an instruction execution system, apparatus, or device, such as a processor. The program code stored in/on the computer readable medium therefore produces an article of manufacture (such as a "computer program product") including program code.

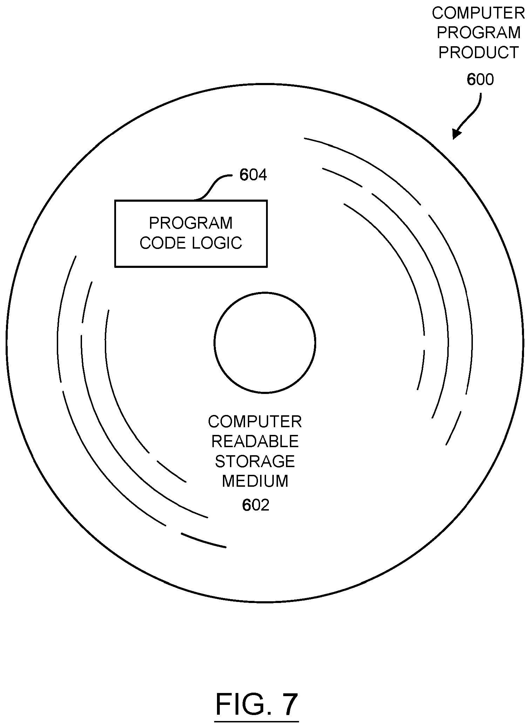

Referring now to FIG. 7, in one example, a computer program product 600 includes, for instance, one or more computer readable media 602 to store computer readable program code means or logic 604 thereon to provide and facilitate one or more aspects of the present invention.

Program code contained or stored in/on a computer readable medium can be obtained and executed by a computer system (computer, computer system, etc. including a component thereof) and/or other devices to cause the computer system, component thereof, and/or other device to behave/function in a particular manner. The program code can be transmitted using any appropriate medium, including (but not limited to) wireless, wireline, optical fiber, and/or radio-frequency. Program code for carrying out operations to perform, achieve, or facilitate aspects of the present invention may be written in one or more programming languages. In some embodiments, the programming language(s) include object-oriented and/or procedural programming languages such as C, C++, C#, Java, etc. Program code may execute entirely on the user's computer, entirely remote from the user's computer, or a combination of partly on the user's computer and partly on a remote computer. In some embodiments, a user's computer and a remote computer are in communication via a network such as a local area network (LAN) or a wide area network (WAN), and/or via an external computer (for example, through the Internet using an Internet Service Provider).

In one example, program code includes one or more program instructions obtained for execution by one or more processors. Computer program instructions may be provided to one or more processors of, e.g., one or more computer system, to produce a machine, such that the program instructions, when executed by the one or more processors, perform, achieve, or facilitate aspects of the present invention, such as actions or functions described in flowcharts and/or block diagrams described herein. Thus, each block, or combinations of blocks, of the flowchart illustrations and/or block diagrams depicted and described herein can be implemented, in some embodiments, by computer program instructions.

The flowcharts and block diagrams depicted and described with reference to the figures illustrate the architecture, functionality, and operation of possible embodiments of systems, methods and/or computer program products according to aspects of the present invention. These flowchart illustrations and/or block diagrams could, therefore, be of methods, apparatuses (systems), and/or computer program products according to aspects of the present invention.

In some embodiments, as noted above, each block in a flowchart or block diagram may represent a module, segment, or portion of code, which includes one or more executable instructions for implementing the specified behaviors and/or logical functions of the block. Those having ordinary skill in the art will appreciate that behaviors/functions specified or performed by a block may occur in a different order than depicted and/or described, or may occur simultaneous to, or partially/wholly concurrent with, one or more other blocks. Two blocks shown in succession may, in fact, be executed substantially concurrently, or the blocks may sometimes be executed in the reverse order. Additionally, each block of the block diagrams and/or flowchart illustrations, and combinations of blocks in the block diagrams and/or flowchart illustrations, can be implemented wholly by special-purpose hardware-based systems, or in combination with computer instructions, that perform the behaviors/functions specified by a block or entire block diagram or flowchart.

It is to be understood that the above description is intended to be illustrative, and not restrictive. Numerous changes and modifications may be made herein by one of ordinary skill in the art without departing from the scope of the invention as defined by the following claims. For example, the above-described embodiments (and/or aspects thereof) may be used in combination with each other. In addition, many modifications may be made to adapt a particular situation or material to the teachings of the various embodiments without departing from their scope. While the dimensions and types of materials described herein are intended to define the parameters of the various embodiments, they are by no means limiting and are merely exemplary. Many other embodiments will be apparent to those of skill in the art upon reviewing the above description. The scope of the various embodiments should, therefore, be determined with reference to the appended claims, along with the full scope to which such claims are entitled.

While several aspects and embodiments of the present invention have been described and depicted herein, alternative aspects and embodiments may be affected by those skilled in the art to accomplish the same objectives. Accordingly, this disclosure and the appended claims are intended to cover all such further and alternative aspects and embodiments as fall within the scope of the invention as defined by the appended claims.

## Claims

1. A computer-implemented method, comprising:
digitally determining an actual position and pose of a known collection of objects from an orthopedic fixation device including spherical fiducial markers in a projected three-dimensional space lying above a digital two-dimensional radiographic space, comprising:
digitally inputting a single first digital radiographic image depicting shadows of the known collection of objects in the projected three-dimensional space lying above the two-dimensional radiographic space;
digitally inputting digital dimensions of the actual external device that include digital dimensions corresponding to actual diameters of the actual fiducial markers and actual distances between the actual fiducial markers;
digitally locating and measuring a measured diameter of the depictions of the fiducial markers and comparing the measured diameter of the depictions of the fiducial markers with the digitally input digital dimensions of the actual diameters of the actual fiducial markers to determine a magnification factor;
utilizing the magnification factor to determine a relative distance between the depictions of the fiducial markers and the actual respective fiducial markers and a relative distance between the depictions of the fiducial markers and an arbitrarily chosen focal point via the magnification factor of each fiducial marker depiction; and
determining the actual position and pose of the known collection of objects in the projected three-dimensional space lying above the two-dimensional radiographic space, using a plurality of closed vector loops through the shadow centers, the centers of the actual portions casting the shadows, and the focal point location of the image.

2. The method of claim 1, further comprising utilizing the actual position and pose of the known collection of objects in the projected three-dimensional space to construct a three-dimensional model of the actual position and pose of the known collection of objects in the projected three-dimensional space.

3. The method of claim 1, further comprising constructing a plurality of closed loop vectors, each closed loop vector extending through a center of one of the actual fiducial markers, a center of one of the depictions of the fiducial markers, and the arbitrarily chosen focal point.

4. The method of claim 3, further comprising solving the closed loop vectors to determine nodal locations of the actual fiducial markers to determine an actual focal point.

5. The method of claim 4, wherein the single first digital radiographic image further comprises a depiction of at least one anatomical structure coupled to the known collection of objects, and further comprising constructing a three-dimensional model of the actual position and pose of the at least one anatomical structure in the projected three-dimensional space.

6. The method of claim 1, wherein the fiducial markers of the orthopedic fixation device include a fiducial marker with a smaller diameter than the other fiducial markers, the other fiducial markers each having the same diameter.

7. The method of claim 1, wherein determining the actual position and pose of the known collection of objects in the projected three-dimensional space lying above the two-dimensional radiographic space comprise constructing a coordinate transformation matrix defining the position and pose of the known collection of objects with respect to the single first digital radiographic image.

8. A computer program product comprising:
a non-transitory computer readable storage medium readable by one or more processing circuit and storing instructions for execution by one or more processor for performing a method according to claim 1.

9. The product of claim 8, wherein the depictions of the fiducial markers define an elliptical shape and measuring the measured diameter of the depictions of the fiducial markers includes measuring a minor diameter of the elliptical shape.

10. The product of claim 9, wherein the single first digital radiographic image further comprises a depiction of at least one anatomical structure coupled to the known collection of objects, further comprising constructing a three-dimensional model of the actual position and pose of the at least one anatomical structure in the projected three-dimensional space, and further comprising determining a relationship between the single first digital radiographic image via comparison of one of the actual fiducial markers that is different than the other actual fiducial markers with a depiction of the fiducial marker that is different than the other depicted fiducial markers.

11. The product of claim 8, wherein determining the actual position and pose of the known collection of objects in the projected three-dimensional space lying above the two-dimensional radiographic space comprise constructing a coordinate transformation matrix defining the position and pose of the known collection of objects with respect to the single first digital radiographic image.

12. A system comprising:
a memory;
at least one processor in communication with memory; and
program instructions executable by one or more processor via the memory to perform a method according to claim 1.

13. The system of claim 12, wherein the depictions of the fiducial markers define an elliptical shape and measuring the measured diameter of the depictions of the fiducial markers includes measuring an average diameter of the elliptical shape.

14. The system of claim 12, wherein the single first digital radiographic image further comprises a depiction of at least one anatomical structure coupled to the known collection of objects, further comprising comparing the measured diameters with the actual diameters between the collection of objects from the orthopedic fixation device to construct a three-dimensional model of the actual position and pose of the at least one anatomical structure in the projected three-dimensional space, and further comprising determining a relationship between the single first digital radiographic image via comparison of a common object of the known collection of objects depicted in the single first digital radiographic image.

15. The system of claim 12, wherein determining the actual position and pose of the known collection of objects in the projected three-dimensional space lying above the two-dimensional radiographic space comprise constructing a coordinate transformation matrix defining the position and pose of the known collection of objects with respect to the single first digital radiographic image.

## Patentansprüche

1. Computerimplementiertes Verfahren, umfassend:
digitales Bestimmen einer tatsächlichen Position und Stellung einer bekannten Sammlung von Objekten aus einer orthopädischen Fixationsvorrichtung einschließlich sphärischer Bezugsmarker in einem projizierten dreidimensionalen Raum, der oberhalb eines digitalen zweidimensionalen radiographischen Raums liegt, umfassend:
digitales Eingeben eines einzelnen ersten digitalen Röntgenbildes, das Schatten der bekannten Sammlung von Objekten in dem projizierten dreidimensionalen Raum, der über dem zweidimensionalen radiographischen Raum liegt, darstellt;
digitales Eingeben digitaler Abmessungen der tatsächlichen externen Vorrichtung, die digitale Abmessungen beinhalten, die den tatsächlichen Durchmessern der tatsächlichen Bezugsmarker und tatsächlichen Abständen zwischen den tatsächlichen Bezugsmarkern entsprechen;
digitales Lokalisieren und Messen eines gemessenen Durchmessers der Darstellungen der Bezugsmarker und Vergleichen des gemessenen Durchmessers der Darstellungen der Bezugsmarker mit den digital eingegebenen digitalen Abmessungen der tatsächlichen Durchmesser der tatsächlichen Bezugsmarker, um einen Vergrößerungsfaktor zu bestimmen;
Verwenden des Vergrößerungsfaktors, um einen relativen Abstand zwischen den Darstellungen der Bezugsmarker und den tatsächlichen jeweiligen Bezugsmarkern und einen relativen Abstand zwischen den Darstellungen der Bezugsmarker und einem willkürlich gewählten Brennpunkt mittels des Vergrößerungsfaktors jeder Bezugsmarkerdarstellung zu bestimmen; und
Bestimmen der tatsächlichen Position und Stellung der bekannten Sammlung von Objekten in dem projizierten dreidimensionalen Raum, der oberhalb des zweidimensionalen radiographischen Raums liegt, unter Verwendung einer Vielzahl von geschlossenen Vektorschleifen durch die Schattenzentren, die Mittelpunkte der tatsächlichen Abschnitte, die die Schatten abwerfen, und den Brennpunktort des Bildes.

2. Verfahren nach Anspruch 1, ferner umfassend Verwenden der tatsächlichen Position und Stellung der bekannten Sammlung von Objekten in dem projizierten dreidimensionalen Raum, um ein dreidimensionales Modell der tatsächlichen Position und Stellung der bekannten Sammlung von Objekten in dem projizierten dreidimensionalen Raum zu erstellen.

3. Verfahren nach Anspruch 1, ferner umfassend Konstruieren einer Vielzahl von geschlossenen Vektorschleifen, wobei sich jede geschlossene Vektorschleife durch einen Mittelpunkt eines der tatsächlichen Bezugsmarker, einen Mittelpunkt einer der Darstellungen der Bezugsmarker und den willkürlich gewählten Brennpunkt erstreckt.

4. Verfahren nach Anspruch 3, ferner umfassend Lösen der geschlossenen Vektorschleifen, um Knotenorte der tatsächlichen Bezugsmarker zu bestimmen, um einen tatsächlichen Brennpunkt zu bestimmen.

5. Verfahren nach Anspruch 4, wobei das einzelne erste digitale Röntgenbild ferner eine Darstellung von mindestens einer anatomischen Struktur umfasst, die mit der bekannten Sammlung von Objekten gekoppelt ist, und ferner umfassend Konstruieren eines dreidimensionalen Modells der tatsächlichen Position und Stellung der mindestens einen anatomischen Struktur in dem projizierten dreidimensionalen Raum.

6. Verfahren nach Anspruch 1, wobei die Bezugsmarker der orthopädischen Fixationsvorrichtung einen Bezugsmarker mit einem kleineren Durchmesser als die anderen Bezugsmarker umfassen, wobei die anderen Bezugsmarker jeweils den gleichen Durchmesser aufweisen.

7. Verfahren nach Anspruch 1, wobei Bestimmen der tatsächlichen Position und Stellung der bekannten Sammlung von Objekten in dem projizierten dreidimensionalen Raum, der über dem zweidimensionalen radiographischen Raum liegt, ein Konstruieren einer Koordinatentransformationsmatrix umfasst, welche die Position und Stellung der bekannten Sammlung von Objekten in Bezug auf das einzelne erste digitale Röntgenbild definiert.

8. Computerprogrammprodukt, umfassend:
ein nichtflüchtiges computerlesbares Speichermedium, das durch eine oder mehrere Verarbeitungsschaltungen lesbar ist und Anweisungen zur Ausführung durch einen oder mehrere Prozessoren zum Durchführen eines Verfahrens nach Anspruch 1 speichert.

9. Produkt nach Anspruch 8, wobei die Darstellungen der Bezugsmarker eine elliptische Form definieren und Messen des gemessenen Durchmessers der Darstellungen der Bezugsmarker ein Messen eines kleinen Durchmessers der elliptischen Form umfasst.

10. Produkt nach Anspruch 9, wobei das einzelne erste digitale Röntgenbild ferner eine Darstellung von mindestens einer anatomischen Struktur umfasst, die mit der bekannten Sammlung von Objekten gekoppelt ist, ferner umfassend Konstruieren eines dreidimensionalen Modells der tatsächlichen Position und Stellung der mindestens einen anatomischen Struktur in dem projizierten dreidimensionalen Raum, und ferner umfassend Bestimmen einer Beziehung bezüglich des einzelnen ersten digitalen Röntgenbildes durch Vergleich eines der tatsächlichen Bezugsmarker, der sich von den anderen tatsächlichen Bezugsmarkern unterscheidet, mit einer Darstellung des Bezugsmarkers, die sich von den anderen dargestellten Bezugsmarkern unterscheidet.

11. Produkt nach Anspruch 8, wobei Bestimmen der tatsächlichen Position und Stellung der bekannten Sammlung von Objekten in dem projizierten dreidimensionalen Raum, der über dem zweidimensionalen radiographischen Raum liegt, ein Konstruieren einer Koordinatentransformationsmatrix, die die Position und Stellung der bekannten Sammlung von Objekten in Bezug auf das einzelne erste digitale Röntgenbild definiert, umfasst.

12. System, umfassend:
einen Speicher;
mindestens einen Prozessor, der mit dem Speicher kommuniziert; und
Programmanweisungen, die von einem oder mehreren Prozessoren über den Speicher ausführbar sind, um ein Verfahren nach Anspruch 1 auszuführen.

13. System nach Anspruch 12, wobei die Darstellungen der Bezugsmarker eine elliptische Form definieren und Messen des gemessenen Durchmessers der Darstellungen der Bezugsmarker ein Messen eines durchschnittlichen Durchmessers der elliptischen Form umfasst.

14. System nach Anspruch 12, wobei das einzelne erste digitale Röntgenbild ferner eine Darstellung von mindestens einer anatomischen Struktur umfasst, die mit der bekannten Sammlung von Objekten gekoppelt ist, ferner umfassend Vergleichen der gemessenen Durchmesser mit den tatsächlichen Durchmessern zwischen der Sammlung von Objekten von der orthopädischen Fixationsvorrichtung, um ein dreidimensionales Modell der tatsächlichen Position und Stellung der mindestens einen anatomischen Struktur in dem projizierten dreidimensionalen Raum zu konstruieren, und ferner umfassend Bestimmen einer Beziehung zu dem einzelnen ersten digitalen Röntgenbild durch Vergleich eines gemeinsamen Objekts der bekannten Sammlung von Objekten, das in dem einzelnen ersten digitalen Röntgenbild dargestellt ist.

15. System nach Anspruch 12, wobei Bestimmen der tatsächlichen Position und Stellung der bekannten Sammlung von Objekten in dem projizierten dreidimensionalen Raum, der über dem zweidimensionalen radiographischen Raum liegt, ein Konstruieren einer Koordinatentransformationsmatrix umfasst, die die Position und Stellung der bekannten Sammlung von Objekten in Bezug auf das einzelne erste digitale Röntgenbild definiert.

## Revendications

1. Procédé mis en œuvre par ordinateur, comprenant :
la détermination numérique d'une position et d'une pose réelles d'une collection connue d'objets à partir d'un dispositif de fixation orthopédique comportant des marqueurs fiduciaires sphériques dans un espace tridimensionnel projeté situé au-dessus d'un espace radiographique bidimensionnel numérique, comprenant :
la fourniture en entrée de manière numérique d'une unique première image radiographique numérique représentant des ombres de la collection connue d'objets dans l'espace tridimensionnel projeté situé au-dessus de l'espace radiographique bidimensionnel ;
la fourniture en entrée de manière numérique de dimensions numériques du dispositif externe réel qui comportent des dimensions numériques correspondant aux diamètres réels des marqueurs fiduciaires réels et aux distances réelles entre les marqueurs fiduciaires réels ;
la localisation et la mesure de manière numérique d'un diamètre mesuré des représentations des marqueurs fiduciaires et la comparaison du diamètre mesuré des représentations des marqueurs fiduciaires avec les dimensions numériques fournies en entrée de manière numérique des diamètres réels des marqueurs fiduciaires réels pour déterminer un facteur d'agrandissement ;
l'utilisation du facteur d'agrandissement pour déterminer une distance relative entre les représentations des marqueurs fiduciaires et les marqueurs fiduciaires respectifs réels et une distance relative entre les représentations des marqueurs fiduciaires et un point focal choisi arbitrairement par l'intermédiaire du facteur d'agrandissement de chaque représentation de marqueur fiduciaire ; et
la détermination de la position et de la pose réelles de la collection connue d'objets dans l'espace tridimensionnel projeté situé au-dessus de l'espace radiographique bidimensionnel, à l'aide d'une pluralité de boucles vectorielles fermées passant par les centres des ombres, les centres des portions réelles projetant les ombres, et l'emplacement du point focal de l'image.

2. Procédé selon la revendication 1, comprenant en outre l'utilisation de la position et de la pose réelles de la collection connue d'objets dans l'espace tridimensionnel projeté pour construire un modèle tridimensionnel de la position et de la pose réelles de la collection connue d'objets dans l'espace tridimensionnel projeté.

3. Procédé selon la revendication 1, comprenant en outre la construction d'une pluralité de vecteurs en boucle fermée, chaque vecteur en boucle fermée s'étendant à travers un centre de l'un parmi les marqueurs fiduciaires réels, un centre de l'une parmi les représentations des marqueurs fiduciaires, et le point focal choisi arbitrairement.

4. Procédé selon la revendication 3, comprenant en outre la résolution des vecteurs en boucle fermée pour déterminer des emplacements nodaux des marqueurs fiduciaires réels afin de déterminer un point focal réel.

5. Procédé selon la revendication 4, dans lequel l'unique première image radiographique numérique comprend en outre une représentation d'au moins une structure anatomique couplée à la collection connue d'objets, et comprenant en outre la construction d'un modèle tridimensionnel de la position et de la pose réelles d'au moins une structure anatomique dans l'espace tridimensionnel projeté.

6. Procédé selon la revendication 1, dans lequel les marqueurs fiduciaires du dispositif de fixation orthopédique comportent un marqueur fiduciaire ayant un diamètre plus petit que celui des autres marqueurs fiduciaires, les autres marqueurs fiduciaires ayant chacun le même diamètre.

7. Procédé selon la revendication 1, dans lequel la détermination de la position et de la pose réelles de la collection connue d'objets dans l'espace tridimensionnel projeté situé au-dessus de l'espace radiographique bidimensionnel comprend la construction d'une matrice de transformation de coordonnées définissant la position et la pose de la collection connue d'objets par rapport à l'unique première image radiographique numérique.

8. Produit de programme informatique comprenant :
un support de stockage non transitoire lisible par ordinateur lisible par un ou plusieurs circuits de traitement et stockant des instructions destinées à être exécutées par un ou plusieurs processeurs pour réaliser un procédé selon la revendication 1.

9. Produit selon la revendication 8, dans lequel les représentations des marqueurs fiduciaires définissent une forme elliptique et la mesure du diamètre mesuré des représentations des marqueurs fiduciaires comporte la mesure d'un diamètre mineur de la forme elliptique.

10. Produit selon la revendication 9, dans lequel l'unique première image radiographique numérique comprend en outre une représentation d'au moins une structure anatomique couplée à la collection connue d'objets, comprenant en outre la construction d'un modèle tridimensionnel de la position et de la pose réelles de l'au moins une structure anatomique dans l'espace tridimensionnel projeté, et comprenant en outre la détermination d'une relation avec l'unique première image radiographique numérique par comparaison de celui parmi les marqueurs fiduciaires réels qui est différent des autres marqueurs fiduciaires réels, avec une représentation du marqueur fiduciaire qui est différent des autres marqueurs fiduciaires représentés.

11. Produit selon la revendication 8, dans lequel la détermination de la position et de la pose réelles de la collection connue d'objets dans l'espace tridimensionnel projeté situé au-dessus de l'espace radiographique bidimensionnel comprend la construction d'une matrice de transformation de coordonnées définissant la position et la pose de la collection connue d'objets par rapport à l'unique première image radiographique numérique.

12. Système comprenant :
une mémoire ;
au moins un processeur en communication avec la mémoire ; et
des instructions de programme exécutables par un ou plusieurs processeurs via la mémoire pour réaliser un procédé selon la revendication 1.

13. Système selon la revendication 12, dans lequel les représentations des marqueurs fiduciaires définissent une forme elliptique et la mesure du diamètre mesuré des représentations des marqueurs fiduciaires comporte la mesure d'un diamètre moyen de la forme elliptique.

14. Système selon la revendication 12, dans lequel l'unique première image radiographique numérique comprend en outre une représentation d'au moins une structure anatomique couplée à la collection connue d'objets, comprenant en outre la comparaison des diamètres mesurés avec les diamètres réels entre la collection d'objets provenant du dispositif de fixation orthopédique afin de construire un modèle tridimensionnel de la position et de la pose réelles de l'au moins une structure anatomique dans l'espace tridimensionnel projeté, et comprenant en outre la détermination d'une relation avec l'unique première image radiographique numérique par comparaison d'un objet commun de la collection connue d'objets représentés dans l'unique première image radiographique numérique.

15. Système selon la revendication 12, dans lequel la détermination de la position et de la pose réelles de la collection connue d'objets dans l'espace tridimensionnel projeté situé au-dessus de l'espace radiographique bidimensionnel comprend la construction d'une matrice de transformation de coordonnées définissant la position et la pose de la collection connue d'objets par rapport à l'unique première image radiographique numérique.
